# EUROPEAN PATENT APPLICATION

(11) **EP 4 821 478 A1**
(43) Date of publication of application: **14.10.2026**
(21) Application number: 26163635.1
(22) Date of filing: 10.03.2026
(51) Int. Cl.: A61J 15/00, A61J 1/10, A61J 1/16, A61J 7/00

(54) **A SYSTEM FOR AGITATING BREAST MILK, A HOUSING WITH AN AGITATION SYSTEM, AND A METHOD FOR AGITATING BREAST MILK**

(30) Priority: 31.03.2025 US 202519095229
(71) Applicant: Medela AG, 6340 Baar (CH)
(72) Inventor: Keclik, James, McHenry, IL 60051 (US)
(74) Representative: Pesce, Michele

(57) **Abstract**

System for providing a newborn with breast milk includes a container to receive breast milk fluidly connected to a tubing system to transport breast milk to the newborn, a dispensing device to convey breast milk from the container through the tubing system to the newborn, and an agitation system to the breast milk. A housing adapted to support a container, the container adapted to receive breast milk and fluidly connected to a tubing system to convey breast milk through a dispensing device to a newborn, the housing including an agitation system to agitate breast milk. Method for providing breast milk to a newborn, wherein a container fluidly connected to a tubing system receives breast milk and conveys breast milk through the tubing system through a dispensing device to the newborn, the breast milk is agitated at least prior to being conveyed through the tubing system to the newborn.

## Description

### FIELD OF THE INVENTION

The present disclosure relates to a system for providing a newborn with breast milk, a housing adapted to receive a cooling system, as well as a method for providing breast milk to a newborn.

### BACKGROUND

Premature infants, i.e. those born before a gestational age of 37 weeks, multiple-birth infants, such as twins or triplets, and other newborns facing early health challenges, typically spend the initial phase of their lives in a neonatal intensive care unit, or "NICU". Here, these newborns are able to gain strength and grow, while being provided with continuous care and monitoring by specifically trained medical staff. However, in most neonatal intensive care units, the newborn is separated from the mother, especially since most mothers are discharged from the corresponding hospital well before their newborn. This presents a practical challenge in providing the breast milk produced by the mother to the newborn. The breast milk must not only be expressed from the mother's breast, but collected, stored, transported, and conveyed to the newborn, all while maintaining the nutritional and safety integrity of said breast milk.

While separated from their newborn, the mother typically expresses and collects the produced breast milk in specific collection receptacles provided with identifying indicia, such as the identity of the mother, the identity of the newborn for whom the breast milk is intended, the volume of breast milk collected, and the time and date of collection, for example. The collected breast milk is subsequently cooled to typical refrigerator temperatures or frozen. After gathering a certain amount of collection receptacles containing breast milk, these are then brought to the hospital or medical clinic for feeding to the newborn. Dependent on the age and medical condition of the newborn, provision of breast milk from the mother to the hospital may occur multiple times a day, daily, every few days, or even weekly. Once received by the medical personnel, the breast milk is then typically stored in a cooled storage facility of the NICU, until required by the newborn.

The general feeding schedule of a newborn requires the conveyance of breast milk to the newborn approximately every three hours. Consequently, the responsible healthcare professional collects the breast milk to be fed to the newborn, confirming that the breast milk corresponds to said newborn and the other identifying indicia are correct. Thereafter, they may prepare a fortified mixture with the breast milk to increase at least one or more of the fat, protein, and carbohydrate contents thereof, prior to transferring the breast milk to a dispensing system operatively and fluidly connected to an enteral feeding pump, for providing the newborn with the breast milk. An enteral feeding pumping is generally required, as the newborn has not yet developed the ability to feed orally, due to the complex suck-swallow-breathe mechanism it has not yet developed. The enteral feeding pump is then manually programmed by the healthcare professional to personalize the required feeding program for the specific newborn being fed. Such a feeding program is typically prescribed and ordered by the attending physician. Once the breast milk has been conveyed to the newborn, the health care professional disengages the newborn from the enteral feeding pump and prepares the enteral feeding pump for use for a different newborn.

Any stagnation phases the breast milk undergoes between expression thereof from the mother's breast to feeding thereof to the newborn, may lead to inconsistencies in the composition and quality of the breast milk. Specifically, if allowed to settle for a certain amount of time, the macronutrients comprising the breast milk make begin to separate, establishing an inhomogeneous breast milk mixture. For example, due to the density differences of macronutrients such as protein, fat, and carbohydrates, distinct separation zones with different concentrations of said macronutrients may be created. Providing a newborn with such an unpredictable composition of breast milk may disrupt the already fragile digestion process of the newborn, ultimately resulting in a longer stay in the NICU.

In an attempt to prevent any separation of the breast milk, the medical staff may continuously check the breast milk during all preparation and feeding steps, mixing the breast milk if deemed necessary at that stage. This intense monitoring and intervention of the breast milk during a rather lengthy and complicated process intensifies not only the tediousness thereof but extends the duration of each feeding session pro newborn.

Therefore, the present invention aims to provide a system for providing a newborn with breast milk, in addition to a corresponding housing and method thereto, that may reduce the risk of breast milk separation by enabling the agitation of the breast milk prior to its conveyance to the newborn, e.g. when the breast milk is contained in the container but is not conveyed to the newborn.

According to a first aspect of the present invention there is provided a system for providing a newborn with breast milk, comprising a container adapted to received said breast milk and fluidly connected to a tubing system to convey the breast milk to the newborn, a dispensing device adapted to convey the breast milk from the container, through the tubing system and to the newborn, and a cooling system including a cooling device adapted to cool the breast milk.

The tubing system conveys the breast milk from the container to the newborn and may physically define the flow path of the breast milk from the container to the newborn. The tubing system may include at least one flexible tube that may be constructed of polyvinyl chloride (PVC), polyurethane, or silicone. The tubing system may be directly coupled with the container. The tubing system may further comprise a nasogastric or orogastric tubing section to be introduced to the newborn's respective anatomy.

The container may be a rigid article, such as commonly associated with a breast milk bottle or syringe dispenser, or a flexible component, such as a breast milk bag or pouch. Preferably, the container is either a breast milk bag or a syringe dispenser. With respect to the latter, a syringe dispenser may include a plunger that tightly fits into a cylindrical barrel of said syringe dispenser; however, a syringe dispenser does not necessarily require a plunger.

The dispensing device is adapted to convey the breast from the container, through the tubing system, and to the newborn. In particular, the dispensing device may comprise a pump, to achieve such conveyance of the breast milk. The dispensing device may provide forces responsible for extracting the breast milk from the container, conveying the breast milk through the tubing system and providing said breast milk to the newborn. The dispensing device may be operatively connected to the tubing system.

The agitation system is adapted to agitate the breast milk, preferably the breast milk received in the container. This may be achieved by inducing the breast milk to flow within the container.

By incorporating the container with breast milk into a system comprising components to convey the breast from said container to the newborn, i.e. the tubing system and the dispensing device, in addition to an agitation system, the breast milk may be agitated, and possibly mixed, prior to being conveyed and fed to the newborn; e.g. the container with the breast milk is agitated while the breast milk is not conveyed through to the newborn. This may help ensure that the breast milk does not separate prior to conveyance thereof to the newborn. Moreover, the agitation system may also counteract any separation process that may have already occurred prior to introduction into the system.

The system may further comprise a control unit for controlling the operation of the agitation system. The control unit may be operatively connected to the agitation system by means of a wired or wireless connection.

Furthermore, the system may also comprise a user interface operatively connected to the control unit. The control unit may be operatively connected to the user interface by means of a wireless or wired connection.

The user interface may be adapted to receive input, preferably from a user, and provide said input to the control unit, in order to control the operation of the agitation system. More specifically, input provided over the user interface may instruct the control unit to set a least one agitation parameter of the agitation system. Preferably, input may be provided over the user interface prior to and during operation of the system, so that the input over the user interface may not only set at least one of the agitation parameters but may adjust at least one of the agitation parameters during use of the system. As such, the control unit may be adapted to set and adjust at least one of the agitation parameters of the agitation system. Therefore, agitation parameters may be modified and adjusted during the conveyance of breast milk from the container, through the tubing system, and to the newborn. Moreover, the user may also set the agitation parameters prior to the operation or use of the system, i.e. prior to breast milk being conveyed from the container.

The user interface may comprise at least one or more of a touch screen, a setting dial, and a press button. Moreover, the user interface may further comprise a display that may display at least one or more of the set agitation parameters, real-time information regarding the agitation parameters, and patient information of the newborn, amongst others.

A agitation parameter is preferably at least or more of an agitation frequency and an agitation duration.

Agitation frequency may be defined as the number of agitation occurrences, such as a stroke, a blow, or an impact, in a given unit of time. An agitation frequency may be between 1 and 60 agitation occurrences per minute, preferably between 20 and 40 agitation occurrences per minute, more preferably 30 agitation occurrences per minute.

An agitation duration may be defined as a time period during which the breast milk is agitated by the agitation system. An agitation duration may be represented as a time interval, such as between 1 and 12 hours, preferably between 2 and 6 hours, more preferably 3 or 4 hours. An agitation duration may also be continuous and uninterrupted. Such a continuous agitation duration may reflect the time period during which the container contains breast milk.

Furthermore, a control unit may also be operatively connected to the dispensing device for controlling the operation of said dispensing device. The control unit may be operatively connected to the dispensing device by means of a wireless or wired connection. Moreover, such a control unit may also be operatively connected to a user interface.

Input provided over a user interface may instruct the control unit to set at least one dispensing parameter of the dispensing device. Preferably, the control unit is also adapted to adjust at least one dispensing parameter of the dispensing device, preferably during use thereof. Consequently, input over the user interface may set and adjust at least one dispensing parameter of the dispensing device during implementation of the dispensing device, preferably during use of the system. This would allow for the use modification and adjustment of dispensing parameters prior to and during the conveyance of breast milk from the container, through the tubing system, to the newborn. Input over the user interface may also set at least one dispensing parameter prior the operation or use of the dispensing device or system.

A dispensing parameter may be at least one or more of a dispensing time and a breast milk amount.

The dispensing time may be defined as the duration during which breast milk is conveyed from the container to the newborn, i.e. a period during which the newborn receives the breast milk. A dispensing time may be between 1 and 60 minutes, preferably between 10 and 50 minutes, more preferably between 20 and 40 minutes and more preferably 30 minutes.

A breast milk amount may be defined as the amount of milk provided to the newborn, which may be associated with a single feeding session. Although preferably given as a volumetric unit of measure, the breast milk amount may also be provided as a mass value.

A user interface operatively connected to the dispensing device may comprise a display to display a least one or more of the set dispensing parameters, real-time information regarding the dispensing parameters, and patient information of the newborn, amongst others.

In a further embodiment of the invention, the system may comprise a housing adapted to support the container. In order to do so, the housing may comprise at least one support on an outer surface of the housing to support the container. Such a support may comprise at least one or more of a bracket, a hook, a peg, and similar mounting system components.

In an alternative embodiment, the housing may exhibit a recess to accommodate the container. The recess may be formed to reflect the general shape of the container, especially the outer boundary contour of the said container, preferably when filled with breast milk. Moreover, the recess may include at least one support, on which the container may be placed or hung, for example. Such a support may carry the weight of the container. Preferably, a support may be realized by a hook, an arm, or a peg, for example.

The housing may further include a cover associated with the recess so that the recess and the cover forms a boundary of a closed compartment adapted to accommodate the container. This may represent a closed position of the cover and the recess. Together, the recess and cover establish an outer boundary such that the container may be fully encompassed by the housing within said closed compartment. Preferably, the shape of the closed compartment reflects the general contour of the container, in particular the general contour of the container with breast milk, preferably filled with breast milk.

The cover, which may be a separate component from the housing, may be attached thereto via a latching system or a hinge or sliding construction. The latter may allow the user to open the closed compartment with one hand, thereby enabling ease of use.

Preferably, when in a closed position, the cover and the recess establish a sealed closed compartment. This may establish a sealed atmospheric environment in the closed compartment.

Preferably, the closed compartment is thermally insulated, especially when in a closed position.

Preferably, the cover may include window or be constructed of a transparent material, such as polycarbonate, Poly(methyl methacrylate) (PMMA), or polyethylene terephthalate (PET). Such a construction allows for a quick visual verification of the fill-status of the container, in addition to other information provided on said container, without the need to open the closed compartment or handle the container during use thereof.

Furthermore, the cover of the housing may include at least one aperture through which the tubing system may pass. Preferably, such an aperture includes a sealing component, such as a sealing ring, in order to ensure that the closed compartment remains sealed against the outside environment of the housing.

In a further embodiment of the system, the agitation system may comprise a re-circulation arrangement including a re-circulation line fluidly connected to the container and a pumping device for circulating the breast milk from the container, through the re-circulation line, and back to the container.

Preferably, the re-circulation line is fluidly connected to the container at an inlet and fluidly connected to the container at an outlet, wherein the breast milk is circulated through the agitation line from the inlet to the outlet. In particular, the inlet of the re-circulation line may be associated with a bottom of the container, whereas the outlet may be may be associated with a top of the container. The re-circulation line may therefore physically define the flow path of the breast milk in the agitation system. The agitation line may include at least on flexible tube that may be constructed of polyvinyl chloride (PVC), polyurethane, or silicone.

Preferably, the pumping device is operatively connected to the control unit by means of a wireless or wired connection, for controlling the operation of said pumping device. Moreover, such a control unit may also be operatively connected to a user interface.

In particular, a user interface may be adapted to receive input, preferably from a user, and provide said input to the control unit, in order to control the operation of the pumping device. More specifically, input provided over the user interface may instruct the control unit to set a least one pumping parameter of the pumping device. Preferably, input may be provided over the user interface prior to and during operation of the system, so that the input over the user interface may not only set at least one of the pumping parameters but may adjust at least one of the pumping parameters during use of the system. As such, the control unit may be adapted to set and adjust at least one of the pumping parameters of the pumping device. Therefore, pumping parameters may be modified and adjusted during the conveyance of breast milk from the container, through the tubing system, and to the newborn. Moreover, the user may also set the pumping parameters prior to the operation or use of the system, i.e. prior to breast milk being conveyed from the container.

Preferably, a pumping parameter may be at least one or more of a breast milk flow rate, and a pumping duration.

A pumping duration may be defined at the time period during which breast milk is circulated through the breast agitation means. A pumping duration may be represented as a time interval, such as between 1 and 12 hours, preferably between 2 and 6 hours, more preferably 3 or 4 hours. A pumping duration may also be continuous and uninterrupted. Such a continuous pumping duration may reflect the time period during which the container contains breast milk.

In a further embodiment of the system, the agitation system may comprise a container agitating means. Such a container agitating means may include an actuator, which may be adapted to cooperate with the container in such a way so as to agitate the breast milk received in said container. As such, the actuator may interact with the container, preferably directly.

The actuator may include at least one reciprocating piston.

The actuator may include at least one rotating cam.

The actuator may include at least one vibrational means to provide vibrational movement to the breast milk received in the container. Preferably, the vibration means is adapted to generate vibrations that interact with the container. In order to do so, the vibration means may be placed in direct contact with the container. This may allow for direct transmission of the vibration forces to the container. However, a further component may be located between the vibrational means and the container, thereby transferring vibration movement from the vibration means to the container and the therein received milk. In such a case, the vibration means may be in direct contact with a support that supports the container, for example.

The container agitating means may comprise at least one or more of a vibration mean, a reciprocating piston and a rotating cam.

Preferably, the container is accommodated in a closed compartment during agitation thereof, in order to agitate the breast milk received therein.

With respect to an embodiment of the invention including a re-circulation arrangement, a housing may preferably support the container and house or contain at least one or more, preferably all, the re-circulation line and the pumping device. More preferably, the housing also accommodates the container.

The agitation system may include both a container agitating means and a re-circulation arrangement. In such an embodiment, at least one of the agitation parameters of the container agitating means may differ from that of the re-circulation arrangement. For example, the agitation duration of the re-circulation arrangement may be continuous, while the agitation duration of the container agitating means may occur in intervals, or vice versa.

Preferably, the system comprises a single user interface operatively connected to at least one, preferably all of, a control unit for controlling the operation of the agitation system, a control unit for controlling the operation a dispensing device, and a control unit for controlling the operation of a pumping device. Preferably, the system comprises a single control unit that is adapted to control the operation of the at least one, more preferably all of, the agitation system, a dispensing device, and a pumping device.

The housing according to any embodiment of the invention may contain or house at least one or more of the agitation system and the dispensing device.

According to a second aspect of the present invention, there is provided a housing adapted to support a container, wherein said container is adapted to receive breast milk and is fluidly connected to a tubing system to convey said breast milk to a newborn. Moreover, the housing is also adapted to receive an agitation system adapted to agitate the breast milk.

According to a third aspect of the present invention, there is provided a method of providing breast milk to a newborn, wherein a container that is fluidly connected to a tubing system receives the breast milk, and wherein said breast milk is conveyed from the container, through the tubing system to the newborn. Moreover, the breast milk received in the container is agitated prior to being conveyed from the container, via the tubing system, to the newborn.

Agitation of the breast milk by the system prior to said breast milk being conveyed to the newborn may refer to the breast milk received in the container being agitated prior to reaching the newborn. Furthermore, conveying breast milk from the container may refer to the extraction or removal of breast milk from said container and the subsequent flow of the breast milk in the direction of the newborn via the tubing system. Therefore, agitating breast milk prior to it being conveyed from the container may refer to the breast milk being agitated before exiting the container. Preferably, the breast milk is agitated prior to entering the tubing system.

Moreover, an amount of breast milk remaining in the container may be agitated while a different amount of breast milk is being conveyed from the container to the newborn. Preferably, breast milk received in the container is agitated independent of an amount of breast milk being conveyed to the newborn. Preferably, the breast milk in the container continues to be agitated while an amount of breast milk is being conveyed from the container through the tubing system to the newborn. As such, the agitation of breast milk received in the container and conveyance of breast milk from the container may occur simultaneously, preferably, independent from one another.

The breast milk received in the container may be agitated continuously, preferably while an amount of breast milk is conveyed from the container to the newborn via the tubing system.

Preferably, an agitation system with a container agitating means may elastically deform the container. This may locally displace the breast milk within the container, resulting in a flow that may prevent separation of the breast milk components or prevent separation thereof via mixing.

However, the agitation system with a container agitating means may also result in movement of the container without the necessity of elastic deformation thereof. The movement of the container may result in movement of the breast milk received therein, thereby resulting in agitation of said breast milk.

In an embodiment of the invention wherein the container agitating means includes two reciprocating pistons, or at least two rotating cams, the movement of said multiple components may be regulated to a-cyclically agitate the breast milk received in the container.

In a further embodiment, the agitation system comprise a re-circulation arrangement that removes the breast milk from the container and transports the breast milk through a re-circulation and back to the container. Preferably, a pumping device pumps the breast milk from the container, through the re-circulation line, and back to the container. Preferably, the agitation line is a re-circulation loop. Moreover, a re-circulation arrangement may also induce flow of the breast milk within the container.

A re-circulation of the breast milk may ensure movement and flow thereof, thereby preventing the stagnation and separation of the breast milk.

Preferably, the container is directly connected to the tubing system. Furthermore, the tubing system is preferably connected to a dispensing device, which ensures that the breast milk is conveyed from the container through the tubing system and to the newborn. The tubing system may be operatively connected to the dispensing device prior to fluidly connecting the tubing system with the container.

This invention may also be said broadly to consist in the parts, elements and features referred to or indicated in the specification of the application, individually or collectively, and any or all combinations of any two or more said parts, elements or features, and where specific integers are mentioned herein which have known equivalents in the art to which this invention relates, such known equivalents are deemed to be incorporated herein as if individually set forth. As used herein "(s)" following a noun could mean the plural and/or singular forms of the noun.

Further details, advantages or features of the present invention will become apparent from the following description in combination with the figures. Examples of embodiments of the invention will be further described with reference to the figures, in which,

### BRIEF DESCRIPTION OF THE SEVERAL VIEWS OF THE DRAWINGS

Figure 1 illustrates a perspective view of a first embodiment of a system;
Figure 2 illustrates an additional perspective view of a first embodiment of a system;
Figure 3 illustrates a perspective view of a second embodiment of a system:
Figure 4 illustrates a side-view of a container on a support;
Figure 5 illustrates a perspective view of an embodiment of a system with a cooling system and an agitation system;
Figure 6 illustrates a side-view of a container accommodated within a housing and connected to a re-circulation arrangement of an agitation system;
Figure 7a illustrates a side-view of a container and a vibration means of a container agitation means accommodated within a housing;
Figure 7b illustrates a side-view of a container and a vibration means of a container agitation means accommodated within a housing;
Figure 8a illustrates a side-view of a container and a reciprocating piston of a container agitation means accommodated within a housing;
Figure 8b illustrates a side-view of a container and two reciprocating pistons of a container agitation means accommodated within a housing;
Figure 9a illustrates a side-view of a container and a rotating cam of a container agitation means accommodated within a housing; and
Figure 9b illustrates a side-view of a container and two rotating cams of a container agitation means accommodated within a housing.

### DETAILED DESCRIPTION OF THE PREFERRED EMBODIMENTS

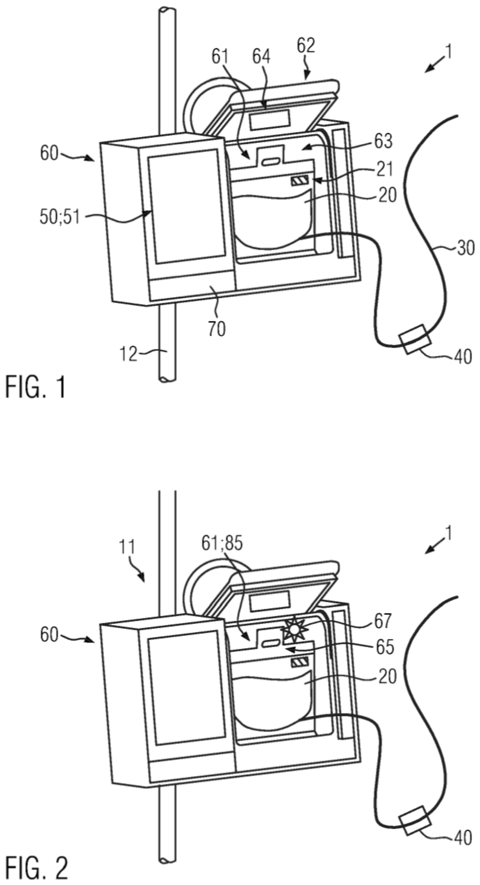

A first embodiment of a system 1, as illustrated in Figures 1 and 2, manifests a container 20 fluidly connected to a tubing system 30 and a dispensing device 40 adapted to convey breast milk from the container 20 through the tubing system 30 to a newborn; e.g. the dispensing device is a pump such as a volumetric pump. A warmer (not shown) may be provided on the tubing system 30, to warm the breast milk to a temperature adapted for feeding the breast milk to the newborn. The container 20, which is depicted as a breast milk bag, is adapted to receive multiple batches of breast milk, while the dispensing device 40 is adapted to provide at least one of said batches of breast milk from said container 20 to the newborn.

The container 20 is supported by support 65 located within a recess 61 of housing 60, wherein said recess 61 is closed by a cover 62 with a window 64. Together, the recess 61 and the cover 62 form a boundary of a closed compartment 63, which surrounds and accommodates the container 20. The window 64 of the cover 62 enables a user to optically review and identify the amount of breast milk within the container 20 without the necessity of opening the cover 62 to the closed compartment 63, in order to do so. Moreover, the cover 62 exhibits an aperture through which the tubing system 30, which is directly connected to the container 20, may pass, allowing for said cover 62 to remain closed during use of the system 1.

The housing 60 of the system 1 further comprises a user interface 51 constructed as a touch screen with a display and operatively connected to a control unit 50, which in turn is operatively connected to the dispensing device 40 for controlling the operation thereof. The user interface 51 allows the provision of input, in order to set and adjust at least one dispensing parameter, such as dispensing time of a batch of breast milk, a breast milk amount of a batch, and an interval time between two subsequent batches, of the dispensing device 40. Moreover, the display of the user interface 51 displays the currently implemented dispensing parameters, as well as further settings, including the conditions of the closed compartment 63 and user data, for example.

Moreover, the system 1 also includes a reader 70 positioned on the housing 60, wherein said reader 70 is adapted to identify an identifier 21 located on the container 20. The identifier 21, which may be realized as a QR-code, NFC tag, or RFID tag, provides the reader 70 with information regarding the breast milk associated with the container 20, in addition to predefined dispensing parameters. Since the reader 70 is operatively connected to the control unit 50, the data provided by the identifier 21 to the reader 70 can directly instruct the control unit 50 without the need to input said parameters via the user interface 51.

The housing 60 of the first embodiment not only exhibits a carrying handle 11 to enable simplified transportation and re-location thereof, but also an attachment means to secure the housing 60 to a stand 12, such as an IV pole. The attachment means is a two-handed clamp mechanism activated by the dual-depression of two buttons, which removes the clamp force from the stand 12 so that the housing 60 may be installed or removed therefrom.

As illustrated in Figure 2, the recess 61 of the housing 60 includes an illumination component 67 that provides light to the closed compartment 63. The illumination component 67 is operatively connected to the user interface 51 and control unit 50, so that the illumination of the closed compartment 63 or the recess 61 can be manually set and adjusted via user input over the user interface 51. Additionally, the illumination component 67 is also operatively connected to the cover 62 in such a way that positioning the cover 62 into an open position, such as that depicted in Figure 2, results in the illumination of the recess 61 and the container 20 accommodated therein.

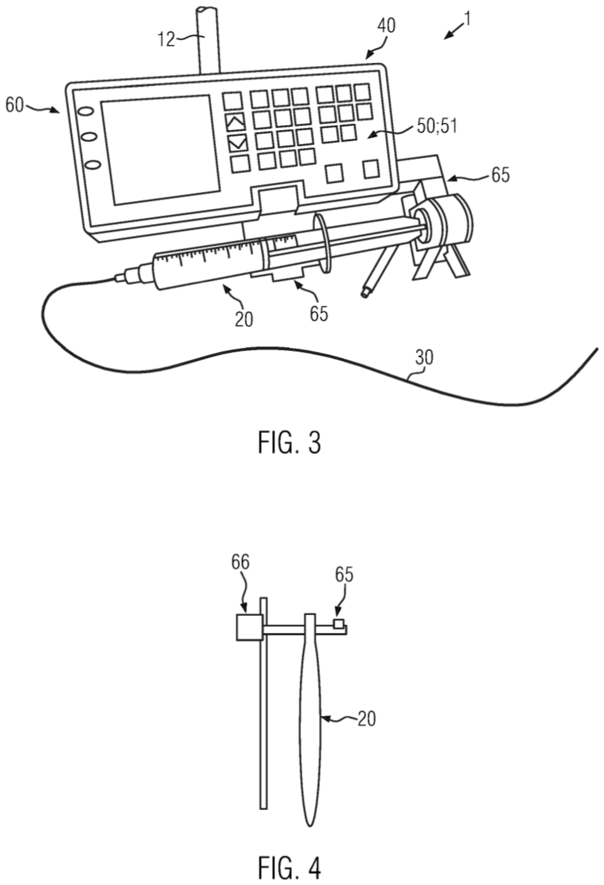

Figure 3 illustrates a second embodiment of the system 1, wherein the supports 65 of the housing 60 that support and accommodate the container 20 project from a bottom side of said housing 60. In said embodiment, the container 20 is realized by a syringe dispenser, which is fluidly connected to the tubing system 30.

As depicted in the embodiment according to Figures 1 and 2, as well as the embodiment of Figure 3, at least two preferably at least four batch amounts of breast milk are provided to and received in the container 20. Subsequently, the container 20 is fluidly connected to the tubing system 30, which is operatively connected to the dispensing device 40, and placed on the supports 65 of the housing 60. Thereafter, the tubing system 30 is fluidly connected to the newborn.

The user than provides input over the user interface 51 regarding the dispensing time of the breast milk batches, a breast milk amount of each batch, and an interval time between two subsequent batches. Subsequently, the control unit 50 instructs the dispensing device 40 accordingly and the breast milk is conveyed from the container 20, through the tubing system 30, and to the newborn in a batch manner. The container 20 remains connected to the tubing system 30 during the conveyance of all of the at least two, preferably at least four, more preferably all, batches of breast milk received in the container 20. Moreover, the container 20 is not refilled during the conveyance of said batches of breast milk or between subsequent batches. The dispensing device 40 retains the remaining batches of breast milk in the container 20 during the interval time of the batched conveyance, so that no breast milk is conveyed from the container 20 or through the tubing system 30 between batched conveyance, i.e. during the interval time between subsequent batches. A valve (not shown) may optionally be provided on the tubing system 30 to retain the remaining batches of breast milk in the container 20 during the interval time of the batched conveyance; e.g. the valve may be provided at the outlet of the container 20 or at the entrance of the dispensing device 40 or at an intermediate position between the two.

Once all batch amounts of breast milk received in the container 20 have been conveyed and provided to the newborn, that is, once the container 20 is empty, then the tubing system 30 is disengaged from the newborn.

The system 1 as illustrated in Figure 4 further comprises a weight-detecting means, wherein the support 65 which upholds the container 20 includes a weight-detecting sensor 66. Although the side-view of the system 1 in Figure 4 depicts the first embodiment according to Figures 1 and 2, it is understood that the second embodiment as illustrated in Figure 3 may also exhibit a weight-detecting means, wherein at least one of the supports 65 is associated with a weight-detecting sensor 66.

The weight-detecting sensor 66, which is operatively connected to the control unit 50 and the user interface 51, continuously measures and records the weight of the container 20. A feedback mechanism in the control unit 50 compares the measured weight value with that of a control value to ensure that the amount of breast milk actually being conveyed by the dispensing device 40 corresponds to the value provided and instructed to the control unit 50. Furthermore, due to the sensitivity of the weight-detecting sensor 66, the system 1 is adapted to detect shock or vibration situations suggesting non-ideal conveying conditions. In such a case, the feedback mechanism of the control unit 50 would instruct the dispensing device 40 to pause further conveyance of the breast milk to the newborn and display and corresponding error message on the display of the user interface 51, so that a user may respond accordingly.

Further embodiments of the system 1 may exhibit a cooling system 80 with a cooling device 81 adapted to cool the breast milk within the container 20 to temperatures between 0 and 15 °C, preferably 1 and 10 °C, more preferably between 2 and 5 °C and e.g. the breast milk is cooled at a temperature of 4 °C. The cooling device 81 in such embodiments may be a heat pump, preferably a thermoelectric heat pump.

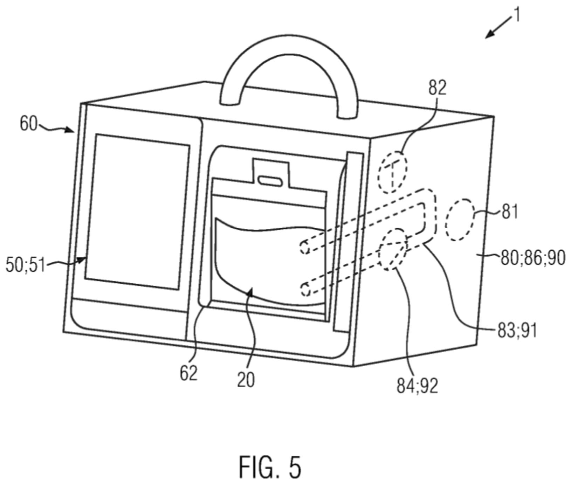

In a third embodiment of the system 1 shown in Figure 5, the cooling system 80 comprises a breast milk cooling means 86 including a cooling line 83 fluidly connected to the container 20, a pumping device 84 for circulating the breast milk from the container 20 through the cooling line 83 to the cooling device 81 and back to the container 20. The cooling device 81 in said embodiment is a thermoelectric heat pump. The individual components of said cooling system 80 are received in the housing 60 of the system 1.

The breast milk is extracted from the container 20 via the pumping device 84 and transported through the cooling line 83 to the cooling device 81, to cool the breast milk. Since the cooling line 83 is realized as a re-circulation loop, the breast milk continues to be transported from the cooling device 81 back to the container 20 via the same pumping device 84.

In a further embodiment of a system 1 including a cooling system 80, as exemplified in Figure 2, said cooling system 80 comprises a compartment cooling means 85 adapted to cool the closed compartment 63 of the housing 60. The closed compartment 63 is preferably thermally insulated. Moreover, by cooling the closed compartment 63, any container 20 with or without breast milk received in said closed compartment 63 would be cooled.

With respect to any embodiment of the invention including the cooling system 80, said cooling system 80 is operatively connected to the control unit 50 so that input over the user interface 51 can instruct the control unit 50 to set at least one cooling parameter of the cooling system 80, such as a cooling temperature and cooling duration. Moreover, in case of an embodiment with a breast milk cooling means 86, input over the user interface 51 can also instruct the control unit 50 to set at least one pumping parameter of the pumping device 84, such as breast milk flow rate and pumping duration. Input provided over the user interface 51 an also be implemented to adjust said parameters during cooling of the breast milk.

The temperature sensor 82 of an embodiment with a breast milk cooling means 86 may be located along the cooling line 83, preferably after the cooling device 81, or in a location associated with the container 20. In case of an embodiment with a compartment cooling means 85, the temperature sensor 82 may be located within the closed compartment 63 of the housing 60, preferably in the recess 61.

As depicted in Figure 5, the breast milk is pumped from the container 20 via the pumping device 84 and transported along the cooling line 83 to the cooling device 81. At the cooling device 81, the breast milk is cooled to a cooling temperature, preferably as instructed by the control unit 51, and then further transported along the remaining cooling line 83 towards the container 20. The transportation of the breast milk through the breast milk cooling means 86 may occur continuously. Specifically, a portion of the breast milk may be transported through the breast milk cooling means 86 while an amount or batch of breast milk is conveyed from the container 20 through the tubing system 30 to the newborn. The breast milk may also be transported through the breast milk cooling means 86 between the conveyance of at least two batches of breast milk.

With respect to an embodiment of the cooling system 80 comprising a compartment cooling means 85, as illustrated in Figures 1 and 2, the closed compartment 63 is cooled when the breast milk is being conveyed from the container 20 and through the tubing system 30 and between the conveyance of at least two batches of breast milk from the container 20, i.e. when no breast milk is conveyed from the container 20.

Additional embodiments of the system 1 may also encompass an agitation system 90 to agitate the breast milk.

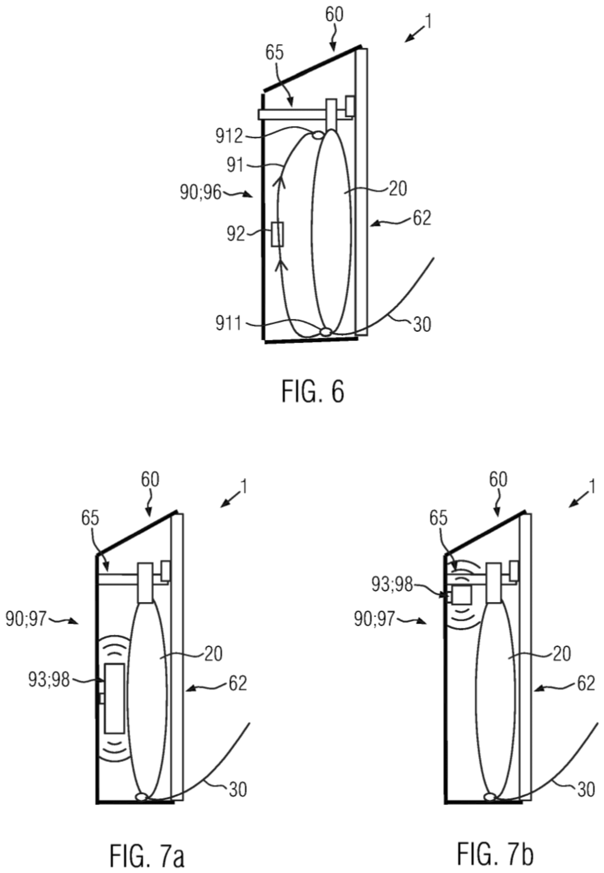

As shown in Figure 6, such an agitation system 90 may comprise a re-circulation arrangement 96, which includes a re-circulation line 91 fluidly connected to the container 20, a pumping device 92 for circulation the breast milk from the container 20 through the re-circulation line 91 and back to the container 20. The re-circulation arrangement 96 may be received in the housing 60 of the system 1.

The re-circulation arrangement 96 provides for the circulation and consequent agitation of the breast milk in the container 20 by extracting the breast milk from the container 20 via the pumping device 92, transporting the breast milk through the re-circulation line 91, and then returning the breast milk to the container 20. Preferably, the breast milk is removed from the container 20 at an inlet 911 of the re-circulation line 91 located at the bottom of the container 20 and returned at an outlet 912 of the re-circulation line 91 associated with the top of the container 20. The re-circulation arrangement 96 also results in the flow of the breast milk within the container 20.

Moreover, the agitation system 90 may comprise a container agitating means 97 that includes an actuator 98 adapted to cooperate with the container 20, in order to agitate the breast milk within said container 20.

Such an actuator 98 may comprise a vibration means 93 adapted to provide vibrational movement to the container 20. As depicted in Figure 7a, the vibration means 93 may be positioned in direct contact to the container 20, in order to ensure direct transmission of the vibrational forces to the breast milk within the container 20. However, the vibration means 93 may be in contact with the support 65 of the housing 60 supporting the container 20, as shown in Figure 7b.

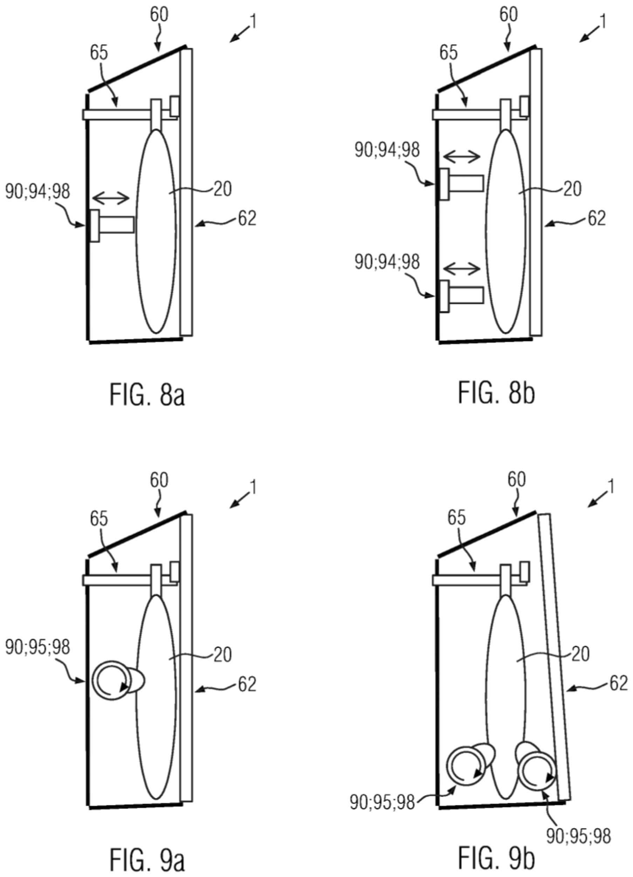

As shown in Figures 8a, 8b, 9a and 9b, the actuator 98 may also comprise at least one reciprocating piston 94 or at least one rotating cam 95. Preferably, the reciprocating piston 94 and the rotating cam 95 elastically deform the container 20 and locally display the breast milk within said container 20, thereby agitating the breast milk within the container 20. However, the reciprocating piston 94 and the rotating cam 95 may simply push on the container 20 without inducing elastic deformation, still ensuring movement of the container 20 and the breast milk therein.

The breast milk is agitated by the agitation system 90 prior to being conveyed from the container 20, through the tubing system 30, and to the newborn. Moreover, the agitation of the breast milk received in the container 20 may occur concurrently to breast milk being conveyed from the container 20, through the tubing system 30, and to the newborn. In particular, a portion of the breast milk may be transported through the re-circulation line 91, while an amount or batch of breast milk is conveyed from the container 20. Breast milk may also be transported through the re-circulation line 91 while no breast milk is being conveyed from the container 20; for example, between batched conveyance thereof. With respect to an embodiment with a container agitating means 97, the container 20 may be agitated, preferably elastically deformed, while an amount or batch of breast milk is conveyed from the container 20. The agitation system 90 may continuously agitate the breast milk received in the container 20.

In a further embodiment, the system 1 may comprise both an agitation system 90 and a cooling system 80, such that the agitation system 90 may encompass a re-circulation arrangement 96 or a container agitating means 97 housed within the closed compartment 63 of the housing, wherein the cooling system 80 comprises a compartment cooling means 85. Such a system 1 may be visualized in Figures 6 to 9b.

Alternatively, a system 1 comprising both an agitation system 90 and a cooling system 80 may exhibit a re-circulation arrangement 96 that may make use of the identical components of the breast cooling means 86. Such an embodiment is illustrated in Figure 5. This prevents the necessity of additional components and allows for the simultaneous cooling and agitation of the breast milk.

With respect to any embodiment of the invention including an agitation system 90, said agitation system 90 is operatively connected to the control unit 50 so that input over the user interface 51 can instruct the control unit 50 to set at least one agitation parameter of the agitation system 90, such as a agitation frequency and agitation duration. Moreover, in case of an embodiment with a re-circulation arrangement 96, input over the user interface 51 can also instruct the control unit 50 to set at least one pumping parameter of the pumping device 92, such as breast milk flow rate, pumping pressure, and pumping duration. Input provided over the user interface 51 an also be implemented to adjust said parameters during agitation of the breast milk.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10 % of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

### List of Reference Signs

- 1: system
- 11: carrying handle
- 12: stand
- 20: container
- 21: identifier
- 30: tubing system
- 40: dispensing device
- 50: control unit
- 51: user interface
- 60: housing
- 61: recess
- 62: cover
- 63: closed compartment
- 64: window
- 65: support
- 66: weight-detecting sensor
- 67: illumination component
- 70: reader
- 80: cooling system
- 81: cooling device
- 82: temperature sensor
- 83: cooling line
- 84: pumping device
- 85: compartment cooling means
- 86: breast milk cooling means
- 90: agitation system
- 91: re-circulation line
- 911: inlet
- 912: outlet
- 92: pumping device
- 93: vibration means
- 94: piston
- 95: cam
- 96: re-circulation arrangement
- 97: container agitating means
- 98: actuator

## Claims

1. A system for providing a newborn with breast milk comprising:
a container adapted to receive said breast milk and fluidly connected to a tubing system to transport the breast milk to the newborn,
a dispensing device adapted to convey the breast milk from the container through the tubing system to the newborn,
the system further comprising an agitation system to agitate the breast milk.

2. The system according to claim 1, further comprising a control unit for controlling the operation of the agitation system and a user interface operatively connected to the control unit,
wherein input over the user interface instructs the control unit to set at least one agitation parameter of the agitation system,
wherein the at least one agitation parameter is at least one of a group including an agitation frequency and an agitation duration.

3. The system according to claim 1, the agitation system comprises a re-circulation arrangement including a re-circulation line which is fluidly connected to the container and a pumping device for circulating the breast milk from the container through the re-circulation line back to the container.

4. The system according to claim 3, an inlet of the re-circulation line is associated with a bottom of the container and an outlet of the re-circulation line is associated with a top of the container.

5. The system according to claim 3, further comprising a housing adapted to support the container and house the re-circulation arrangement.

6. The system according to claim 3, further comprising a control unit for controlling the operation of the re-circulation arrangement and a user interface operatively connected to the control unit,
wherein input over the user interface instructs the control unit to set at least one pumping parameter of the pumping device,
wherein the at least one pumping parameter is at least of group including a breast milk flow rate and pumping duration.

7. The system according to claim 1, wherein the agitation system comprises an actuator adapted to agitate the breast milk in the container, including at least one of a group including a vibration means adapted to provide vibrational movement to the container, a reciprocating piston, and a rotating cam.

8. The system according to claim 7, further comprising a housing adapted to support the container and house the container agitating means.

9. The system according to claim 1, the container adapted to receive multiple batches of breast milk.

10. A housing adapted to support a container, the container adapted to receive breast milk and being fluidly connected to a tubing system to convey said breast milk through a dispensing device to a newborn,
the housing having an agitation system to agitate the breast milk.

11. A method for providing a breast milk to a newborn,
wherein a container that is fluidly connected to a tubing system receives the breast milk and conveys said breast milk through the tubing system through a dispensing device to the newborn,
the breast milk in the container is agitated at least prior to being conveyed through the tubing system to the newborn.

12. The method according to claim 11, wherein a container agitating means elastically deforms the container to locally displace the breast milk within the container.

13. The method according to claim 11, wherein a re-circulation arrangement induces breast milk flow in the container.

14. The method according to claim 11, wherein the container is agitated while the breast milk is not conveyed through the tubing system to the newborn.

15. The method according to claim 11, wherein the container is adapted to receive multiple batches of breast milk.
